# EUROPEAN PATENT APPLICATION

(11) **EP 4 265 273 A1**
(43) Date of publication of application: **25.10.2023**
(21) Application number: 21909197.2
(22) Date of filing: 13.12.2021
(51) Int. Cl.: A61K 47/60, A61K 47/54, A61K 39/44, A61P 35/00

(54) **ANTIBODY CONJUGATE AND METHOD FOR ENHANCING IMMUNE EFFECT FUNCTION OF ANTIBODY MOLECULE**

(30) Priority: 21.12.2020 CN 202011516850
(71) Applicant: Jiangnan University, Wuxi, Jiangsu 214000 (CN)
(72) Inventor: WU, Zhimeng, Wuxi, Jiangsu 214000 (CN); HONG, Haofei, Wuxi, Jiangsu 214000 (CN); ZHOU, Kun, Wuxi, Jiangsu 214000 (CN); ZHOU, Zhifang, Wuxi, Jiangsu 214000 (CN)
(74) Representative: Osterhoff, Utz
(86) International application number: PCT/CN2021/137352
(87) International publication number: WO 2022/135200

(57) **Abstract**

Antibody conjugates with enhanced immune effector functions of antibody, are disclosed herein. The antibody conjugates are obtained by modifying the antibody with at least a hapten derivative. The hapten derivative consists of a hapten, a linker, and a coupling domain. Through a mild and simple method, the hapten derivative is conjugated to the antibody, resulting in an antibody conjugate that not only retains the excellent affinity of the original antibody but also recruits specific natural antibodies to target tumor cells. This antibody conjugate has enhanced immune effector functions such as antibody-dependent cell-mediated cytotoxicity (ADCC) and complement-dependent cytotoxicity (CDC). Importantly, the present invention optimizes the length of the linker and develops SMCC-PEG3-Rha as the hapten derivative. Compared to other reported antibodies, the antibody conjugate modified with SMCC-PEG3-Rha exhibits more prominent immune effector functions.

## Description

### BACKGROUND OF THE INVENTION :

### 1. Technical Field

Technical Field: The present invention pertains to the field of pharmaceutical engineering and specifically relates to an antibody conjugate and a method for enhancing antibody molecular immune effector functions.

### 2. Description of Related Art

Malignant tumors continue to pose a major threat to human health, with both the incidence and mortality rates on the rise. Monoclonal antibodies are among the most effective biopharmaceuticals used in anti-cancer therapy. They exert therapeutic effects by targeting tumor-specific antigens or related antigens, primarily through mechanisms such as inducing or blocking cell signaling, activating immune effector functions, and serving as carriers for specific drug delivery to target cells. Among these mechanisms, immune effector functions, including antibody-dependent cellular cytotoxicity (ADCC) and complement-dependent cytotoxicity (CDC), play a crucial role and represent the fundamental functionality of most anti-tumor antibody drugs. Therefore, enhancing the ADCC and CDC activities of antibodies through antibody engineering has become a hot topic in antibody biopharmaceutical research and development.

Currently, antibody engineering techniques are predominantly used to enhance antibody ADCC and CDC activities, leading to the development of next-generation monoclonal antibodies with improved immune activities. However, these techniques are complex and require extensive screening to obtain antibodies with satisfactory immunoreactivity, resulting in significant workloads. Moreover, modified antibodies exhibit a certain degree of immunogenicity, leading to immune-related side effects.

There are naturally occurring antibodies in the human body that are specific to certain haptens. Examples of such antibodies with relatively high abundance include antibodies against Rha (rhamnose), DNP (2,4-dinitrophenol), and αGal (alpha-galactose). Modifying commercial monoclonal antibodies with these haptens holds promise for enhancing the immune effector functions of the original antibodies. For instance, it has been reported that conjugating αGal derivatives to anti-CD20 antibodies can yield CD20 antibodies with enhanced CDC levels. However, this approach is hindered by complex modification methods that can affect antibody affinity, product stability, and yield. Additionally, it fails to assess the ADCC levels of the resulting antibodies and optimize the length of critical factors such as the linker, ultimately limiting the improvement of immune effector function levels.

Therefore, there is an urgent need for a novel method to enhance antibody immune effector functions, addressing or mitigating the aforementioned issues.

**Summary of the Invention:** The purpose of the present invention is to overcome the limitations of existing technologies. It provides an antibody conjugate and a method for enhancing immune effector functions of antibody, based on a strategy involving the recruitment of naturally occurring hapten-specific antibodies. Furthermore, by optimizing the linker, the invention offers an optimized selection of hapten derivative molecules that result in antibody conjugate with significantly improved immune effector functions compared to previously reported antibodies.

### Technical Solution:

To achieve the aforementioned objectives, the present invention employs the following technical solutions:
In some aspects, the present disclosure provides an antibody conjugate, comprising an antibody modified by a hapten derivative molecule. The hapten derivative molecule consists of a hapten, a linker, and a coupling domain.

In some aspects, the present disclosure provides the antibody conjugate, wherein the hapten is a small molecule capable of recruiting specific natural antibodies in the human body. Examples of such haptens include, but are not limited to, Rha, DNP, and αGal.

In some aspects, the present disclosure provides the antibody conjugate, wherein the linker comprises, but is not limited to, a polyethylene glycol linker, alkyl chain or aliphatic chain linker or spacer ((CH₂)ₙ), and a peptide linker or spacer.. In some aspects, to facilitate the synthesis of homogeneous products and minimize undesired toxic side effects, the preferred linker arm comprises, but is not limited to, a PEG with low molecule weight (less then 1000), a short alkyl chain or aliphatic chain linker (molecular weight less than 100) and a peptide linker, and further, in some embodiments, low polyethylene glycol PEGs is preferred.

In some aspects, the coupling domain of the invention is used to modify hapten derivatives on antibodies.

In some embodiments, in order to facilitate the synthesis of uniform products and reduce the impact of hapten derivatives on the original affinity of antibodies, the coupling site is preferably the eight thiol groups formed by the reduction of four disulfide bonds on the antibodies; In some embodiments, the coupling domain is preferably SMCC, or other molecules containing maleimides 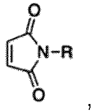 or other molecules containing vinyl sulfones 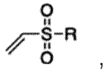 or other molecules containing acrylates or acrylamides 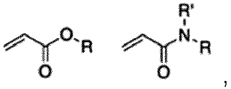 or other molecules containing methacrylates, 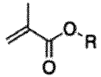 , R and R' is one or more substituents.

In some aspects, the present disclosure provides the antibody conjugate, wherein the antibody is a monoclonal antibody, including but not limited to anti-CD20 antibody, anti-CD19 antibody, anti-CD30 antibody, anti-EGFR antibody, anti-EGFRvIII antibody, anti-HER2 antibody, anti-HER3 antibody, anti-PSMA antibody, anti-VEGFR antibody, anti-PD-L1 antibody, anti-cMET antibody, anti-TGF-β antibody, anti-MUC1 antibody, and anti-Trop-2 antibody.

In some embodiments, the present disclosure provides the antibody conjugate, wherein the heavy chain of antibody is represented by SEQ ID NO.1, and the light chain of antibody is represented by SEQ ID NO.2.

In some embodiments, the present disclosure provides the antibody conjugate, wherein the hapten derivative includes one of the following structures:

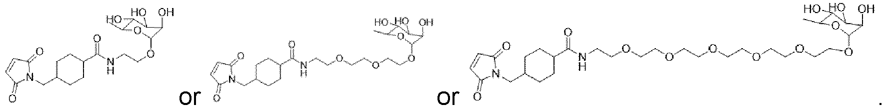

In some embodiments, the present disclosure provides the antibody conjugate, wherein the synthetic pathway of the hapten derivative is: 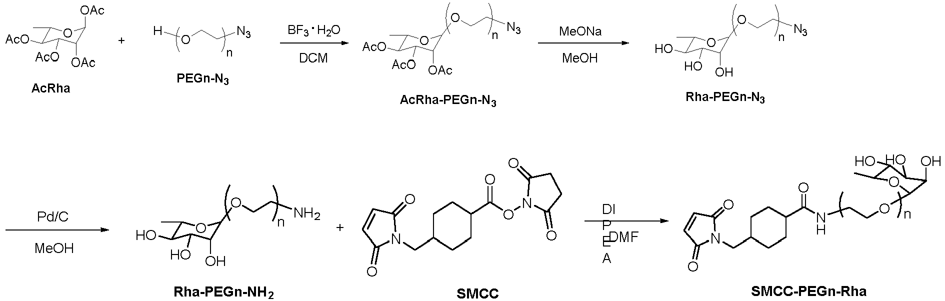 wherein n is a positive integer.

Another objective of the present invention is to provide a method for systhesizing the antibody conjugate with enhanced immune effector functions of antibodies by modifying antibodies with hapten derivative molecules, thereby utilizing the recruitment ability of naturally occurring antibodies specific to the hapten molecules to enhance immune activities such as ADCC and CDC. Furthermore, the invention aims to optimize the linker of the hapten derivative molecules to obtain antibody conjugates with significantly improved immune effector functions.

The hapten derivative molecules, antibodies, modification sites, and methods for enhancing the molecular immune effector functions of antibodies are as previously described.

In an embodiment of the present invention, a method for preparing the antibody conjugate by modifying the hapten derivative onto the antibody, comprising the following steps:
1) Desalting the antibody in a sodium borate reaction.
2) Adding a thiol-containing reducing agent TCEP and reacting under dark and shaking conditions.
3) After the reaction is completed, adding a 10-fold excess of the hapten derivative and quenching the reaction with cysteine.
4) Ultrafiltration to remove excess small molecules and obtain the corresponding antibody conjugate.

In a specific embodiment of the present invention, the selected antibody is rituximab, a monoclonal antibody against CD20 (KEGG Accession Number: DB00073). The heavy chain is represented by SEQ ID NO.1, and the light chain is represented by SEQ ID NO.2. CD20 is highly expressed on the surface of over 95% of B-cell lymphoma cells, making it an important target for monoclonal antibody-based therapies. The optimized hapten derivative molecule is SMCC-PEG3-Rha 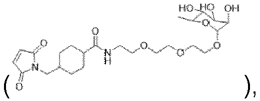 where SMCC represents the coupling domain, PEGs represents a polyethylene glycol chain, and Rha represents the hapten. The coupling site is the eight thiol groups formed after reducing the four disulfide bonds on rituximab antibody.

### Beneficial effects: The advantages of the present invention compared to prior art:

(1) The antibody conjugates described in the present invention exhibit both unimpaired antigen affinity levels compared to reported antibodies and significantly enhanced ADCC and CDC activities. This allows for lower dosages during treatment, ultimately reducing or avoiding the occurrence of certain toxic side effects.
(2) The optimized hapten derivative molecules provided in the present invention lead to antibody conjugates with the most prominent immune effector functions. These conjugates demonstrate significantly higher CDC killing effects compared to wild-type antibodies, using only 4% of the dosage of the wild-type antibody.
(3) The strategy employed in the present invention to enhance antibody immune effector functions is not only simple to implement and utilizes mild modification conditions but also avoids potential toxicity and immunogenicity by harnessing the body's own naturally occurring antibodies.

### Brief Description of Figures

Figure 1 provides the MS of the light chains of rituximab and rituximab conjugates α CD20-PEG₁-Rha, αCD20-PEG₃-Rha and αCD20-PEG₆-Rha.
Figure 2 provides the flow cytometry of cells treated with PBS (negative control), rituximab, or rituximab conjugatesαCD20-PEG₁-Rha, αCD20-PEG₃-Rha or α CD20-PEG₆-Rha. (A) binding affinity assay; (B) antibody-recruiting ability assay; MFI: mean fluorescence intensity.
Figure 3 provides the ADCC mediated by different concentrations of rituximab or rituximab conjugates αCD20-PEG₁-Rha, αCD20-PEG₃-Rha or αCD20-PEG₆-Rha. (A) in the absence of anti-Rha antibodies; (B) in the presence of anti-Rha antibodies.
Figure 4 provides the CDC mediated by different concentrations of rituximab or rituximab conjugates αCD20-PEG₁-Rha, αCD20-PEG₃-Rha or αCD20-PEG₆-Rha. (A) in the absence of anti-Rha antibodies; (B) in the presence of anti-Rha antibodies.

### Detailed Description of the Embodiments

The present invention discloses a class of antibody-hapten conjugates, and a method to magnify the immunity effector functions of antibody. Antibody-hapten conjugates are antibody molecules modified with hapten derivates, and hapten derivates are synthesized small molecules capable of binding to the existing antibodies in human serum. The present invention provides a simple and east method to generate antibody-hapten conjugates. Theses conjugates retain excellent binding specificity and binding affinity, and are able to recruit the existing antibodies onto the cancer cell surface and further form an immune complex that is able to provide multivalent Fc domains to interact with immune cells or complement protein C1q, leading to magnified ADCC and CDC simultaneously. Moreover, the present invention also provides conjugates with optimal ADCC and CDC activities according to the structure-activity relationship. The following embodiments are used to describe the present presentation. However, they are not intended to limit the scope of the present invention. Any simple improvement under the conception of the present invention is within the scope of protection of the present invention. All experimental methods in the following embodiments are conducted according to the procedures provide by companies unless otherwise stated. All the materials and cell lines in the following embodiments are purchased commercially unless otherwise stated.

### Embodiment

In the present embodiments, SMCC, PEG and Rha are selected as the coupling domain, the linker and the hapten, respectively. According to the synthesis methods of the antibody-hapten conjugates in the present invention, the hapten derivatives SMCC-PEGn-Rha (n=1, 3, 6) are firstly synthesized. Then, hapten derivates are coupled to the antibody molecules to generate the corresponding antibody conjugates. Notably, the structure-activity relationship between the length of PEG linker and the biological activities of conjugates was evaluated.

The details, including the coupling domain, the linker and the hapten in the following embodiments, are used to describe the present presentation. However, they are not intended to limit the scope of the present invention.

### Embodiment 1: Chemical Synthesis of Hapten Derivatives SMCC-PEGn-Rha Containing Different PEG Linkers

(1) The synthesis procedure of hapten derivate SMCC-PEG₁-Rha is as follows: 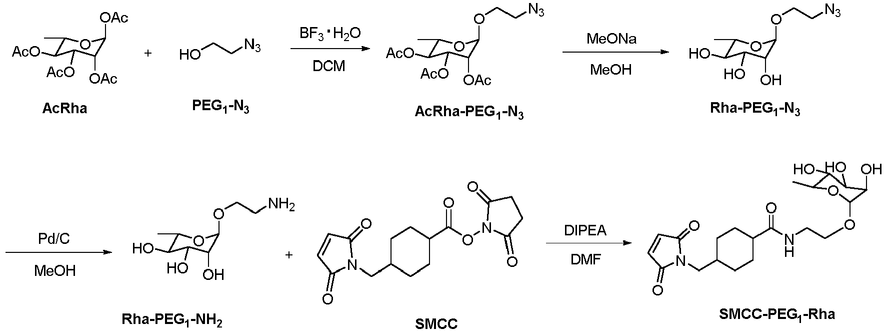 The observed ¹H-NMR, ¹³C NMR and HRMS of SMCC-PEG₁-Rha are all in line with the calculated theoretical date. SMCC-PEG₁-Rha: ¹H NMR (400 MHz, Methanol-d4) δ 6.80 (s, 2H), 4.66 (d, J = 1.7 Hz, 1H), 3.79 (dd, J = 3.5, 1.7 Hz, 1H), 3.69 (dt, J = 10.0, 5.7 Hz, 1H), 3.63 (dd, J = 9.5, 3.5 Hz, 1H), 3.58 - 3.51 (m, 1H), 3.45 (ddd, J = 10.3, 6.1, 4.7 Hz, 1H), 3.40 - 3.31 (m, 6H), 2.14 (tt, J = 12.2, 3.5 Hz, 1H), 1.82 (dd, J = 13.5, 3.4 Hz, 2H), 1.76 - 1.69 (m, 2H), 1.42 (qd, J = 13.1, 3.3 Hz, 2H), 1.24 (d, J = 6.2 Hz, 3H), 1.00 (qd, J = 13.1, 3.5 Hz, 2H). ¹³C NMR (101 MHz, Methanol-d4) δ 177.68, 171.37, 133.87, 100.27, 72.53, 70.94, 70.75, 68.48, 65.55, 44.70, 43.12, 38.74, 36.49, 29.58, 28.66, 16.60. HRMS (ESI, positive) calculated for C₂₀H₃₀N₂O₈Na [M+Na]⁺: 449.1900 Found: 449.1899.
(2) The synthesis procedure of hapten derivate SMCC-PEG₃-Rha is as follows: 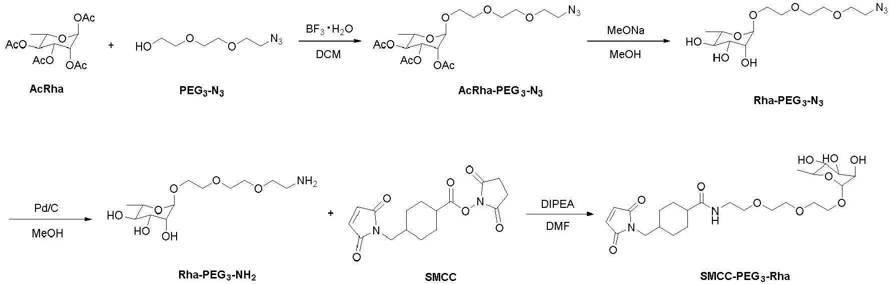 The observed ¹H-NMR, ¹³C NMR and HRMS of SMCC-PEG₃-Rha are all in line with the calculated theoretical date. SMCC-PEG₃-Rha:¹H NMR (400 MHz, Methanol-d4) δ 6.71 (s, 2H), 4.62 (d, J = 1.7 Hz, 1H), 3.73 - 3.65 (m, 2H), 3.57 - 3.49 (m, 8H), 3.43 (t, J = 5.5 Hz, 2H), 3.30 - 3.19 (m, 6H), 2.05 (tt, J = 12.1, 3.4 Hz, 1H), 1.72 (dd, J = 13.5, 3.5 Hz, 2H), 1.66 - 1.60 (m, 2H), 1.38 - 1.20 (m, 3H), 1.16 (d, J = 6.2 Hz, 3H), 0.91 (qd, J = 12.9, 3.5 Hz, 2H). ¹³C NMR (101 MHz, Methanol-d4) δ 133.88, 100.41, 72.59, 70.99, 70.82, 70.26, 70.05, 69.27, 68.41, 66.30, 48.27, 48.05, 47.84, 47.63, 47.41, 47.20, 46.99, 44.67, 43.14, 38.86, 29.57, 28.63. HRMS (ESI, positive) calculated for C₂₄H₃₈N₂O₁₀Na [M+Na]⁺: 537.2424 Found: 537.2415.
(3) The synthesis procedure of hapten derivate SMCC-PEG₆-Rha is as follows: 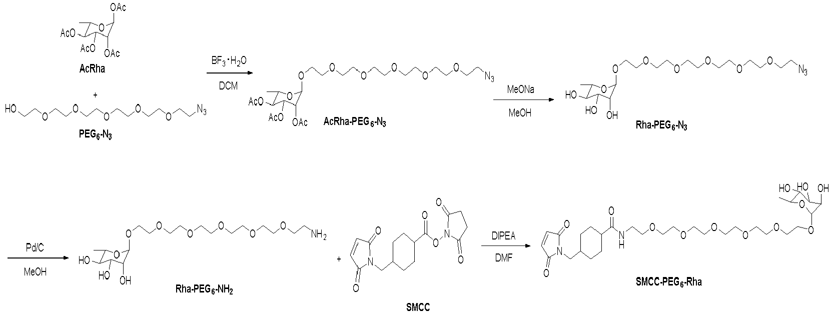 The observed ¹H-NMR, ¹³C NMR and HRMS of SMCC-PEG₆-Rha are all in line with the calculated theoretical date. SMCC-PEGs-Rha: ¹H NMR (400 MHz, Methanol-d4) δ 6.81 (s, 2H), 4.71 (d, J = 1.7 Hz, 1H), 3.82 - 3.75 (m, 2H), 3.68 - 3.57 (m, 22H), 3.52 (t, J = 5.5 Hz, 2H), 3.40 - 3.31 (m, 5H), 2.15 (tt, J = 12.2, 3.6 Hz, 1H), 1.82 (dd, J = 13.7, 3.5 Hz, 2H), 1.76 - 1.69 (m, 2H), 1.42 (qd, J = 13.1, 3.3 Hz, 2H), 1.25 (d, J = 6.2 Hz, 3H), 1.01 (qd, J = 12.9, 3.6 Hz, 2H). ¹³C NMR (101 MHz, Methanol-d4) δ 171.39, 133.89, 100.43, 72.61, 70.98, 70.81, 70.28, 70.22, 70.20, 70.18, 70.16, 70.13, 70.02, 69.88, 69.19, 68.41, 66.40, 44.67, 43.14, 38.86, 36.51, 29.60, 28.63, 16.67. HRMS (ESI, positive) calculated for C₃₀H₅₁N₂O₁₀ [M+H]⁺: 647.3391 Found: 647.3389.

### Embodiment 2: Preparation of Rituximab-Rha conjugates

The procedures for preparing rituximab-Rha conjugates are as follows:
1) Rituximab was exchanged into 25 mM of sodium borate buffer.
2) Tris(2-carboxyethyl)phosphine (TCEP) was added to break the four pairs of disulfide bonds in dark, the reaction progress was monitored by Ellman's analysis.
3) The reduced Rituximab was conjugated with 10 equiv of SMCC-PEG1-Rha, SMCC-PEG₃-Rha, or SMCC-PEG₆-Rha, then the reaction was quenched using cystine.
4) Excess hapten derivates were removed by ultrafiltration to give the final rituximab-Rha conjugates αCD20-PEG₁-Rha, αCD20-PEG₃-Rha and αCD20-PEG₆-Rha.

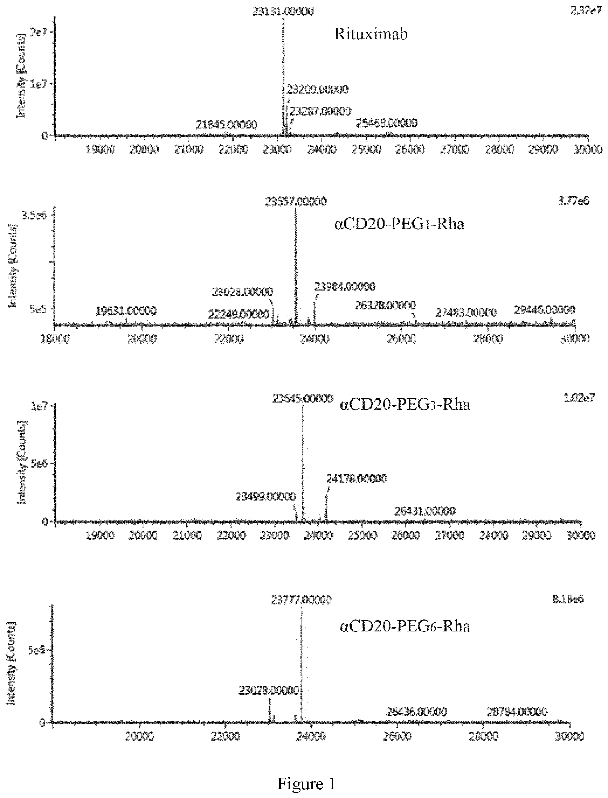

The conjugates were further characterized by LC-MS analysis. As shown in Figure 1, the observed MS of the light chains of rituximab and rituximab conjugates αCD20-PEG₁-Rha, αCD20-PEG₃-Rha and αCD20-PEG₆-Rha were 23131, 23557, 23645 and 23777 Da, respectively, all of which were in line with their calculated molecular weight.

### Embodiment 3: The Binding Affinity and Antibody-Recruiting Ability of Rituximab-Rha Conjugates

The procedure of flow cytometry is as follows:
1) Raji cells (CD20 positive) and K562 cells (CD20 negative) were collected and resuspended to 4×10⁵/mL. Then, 100 µL of these cells was added into tubes containing 50 nM of antibody samples. The incubation was conducted on ice for 30 min.
2) Cells were incubated with FITC-conjugated goat anti-human IgG antibodies (for binding affinity assay), or successively treated with 1% anti-Rha rabbit serum and FITC-conjugated goat anti-rabbit IgG antibodies (for antibody-recruiting ability assay).
3) Cells were finally detected using the Accuri C6 flow cytometer.

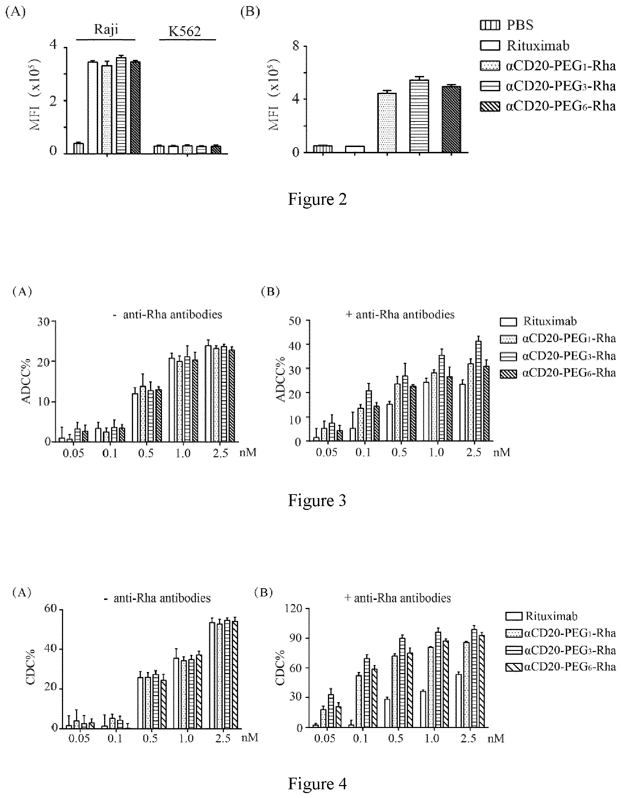

As shown in Figure 2A, for Raji cells treated with rituximab, or conjugates αCD20-PEG₁-Rha, αCD20-PEG₃-Rha or αCD20-PEG₆-Rha, all showed significantly increased fluorescence signals. By contrast, no increased fluorescence signal was observed on K562 cells, suggesting that rhamnosylation did not impair the structure of rituximab significantly and retained excellent binding specificity and binding affinity to CD20 positive cells.

The results of antibody-recruiting ability were presented in Figure 2B, compare to negative control, only Raji cells treated with conjugates showed significantly increased fluorescence signals, indicating that rituximab after rhamnosylation could successfully redirect anti-Rha antibodies onto CD20 positive cells. Figure 2B also proved conjugate αCD20-PEG₃-Rha existed the highest recruiting ability for anti-Rha antibodies.

### Embodiment 4: The ADCC and CDC assays of Rituximab-Rha Conjugates

The ADCC and CDC mediated by rituximab-Rha conjugates in the absence or presence of anti-Rha antibodies were all evaluated.

ADCC assay: 1) Raji cells were collected and resuspended to 4×10⁵/mL. Then, 50 µL of these cells was added to each well in 96-well plates. 2) Immediately supplemented with 50 µL different concentrations of antibody samples (or PBS as the negative control), 50 µL of 4% anti-Rha antibodies and 50 µL of freshly isolated human peripheral blood mononuclear cells (PBMCs), the incubation was performed at 37 °C for 4 h. 3) The cytotoxicity was determined using an LDH kit.

ADCC results were displayed in Figure 3. Rituximab and conjugates presented parallel ADCC activities when in the absence of anti-Rha antibodies (Figure 3A). For example, the lysis rates of cells treated with 2.5 nM of rituximab or conjugates αCD20-PEG₁-Rha, αCD20-PEG₃-Rha or αCD20-PEG₆-Rha, were all around 20%. Whereas when in the presence of anti-Rha antibodies (Figure 3B), all three conjugates showed enhanced ADCC activity, with lysis rates ranging from 30% to 42%. This result suggested that the method in the present invention could successfully magnify the ADCC function of antibody. Notably, conjugate αCD20-PEG₃-Rha existed the highest ADCC activity, the percentage of cell lysis could reach 41.3% when the final concentration of αCD20-PEG₃-Rha was 2.5 nM.

CDC assay: 1) Raji cells were collected and resuspended to 4×10⁵/mL. Then, 50 µL of these cells was added to each well in 96-well plates. 2) Immediately supplemented with 50 µL different concentrations of antibody samples (or PBS as the negative control), 50 µL of 4% anti-Rha antibodies and 50 µL of 8% rabbit total complement, the incubation was performed at 37 °C for 4 h. 3) The cytotoxicity was determined using an LDH kit.

CDC results were displayed in Figure 4. Rituximab and conjugates presented parallel CDC activities when in the absence of anti-Rha antibodies (Figure 4A). For example, the lysis rates of cells treated with 2.5 nM of rituximab or conjugates αCD20-PEG₁-Rha, αCD20-PEG₃-Rha or αCD20-PEG₆-Rha, were all around 50%. Whereas when in the presence of anti-Rha antibodies (Figure 4B), all three conjugates showed enhanced CDC activity, with lysis rates ranging from 85% to 100%. This result suggested that the method in the present invention could successfully magnify the CDC function of antibody. Notably, conjugate αCD20-PEG₃-Rha existed the highest CDC activity, when cells treated with only 0.1 nM of αCD20-PEG₃-Rha, the percentage of cell lysis could reach 69.3%, which is significantly higher than cells treated with 2.5 nM of Rituximab (53.2%). This results also suggested that in the presence of anti-Rha antibodies, the dosage of conjugate αCD20-PEG₃-Rha could be dramatically reduced when compared to primary rituximab.

According to the above embodiments, rituximab-Rha conjugate αCD20-PEG₃-Rha existed the highest ADCC and CDC activities, in another words, has made a tremendous improvement for ADCC and CDC. By contrast, conjugate αCD20-PEG₁-Rha containing a shorter PEG linker and αCD20-PEG₆-Rha containing a longer PEG linker both showed lower ADCC and CDC activities. This difference may be explained by the steric hindrance which limit the interaction of αCD20-PEG₁-Rha and anti-Rha antibodies, resulting in the lower antibody-recruiting level. On the other hand, this difference may be also explained by the unexpected reduced affinity of αCD20-PEG₆-Rha against anti-Rha antibodies, resulting in restricted ADCC and CDC activities.

The above embodiments are used to describe the present presentation. However, they are not intended to limit the scope of the present invention. Any simple improvement under the conception of the present invention is within the scope of protection of the present invention.

## Claims

1. An antibody conjugate, formed by modifying an antibody with at least one hapten derivative, wherein:
the antibody is a monoclonal antibody;
the hapten derivative is composed of a hapten, a linker, and a coupling domain;
the hapten derivative includes one of the following structures:

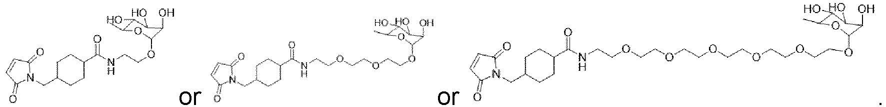

2. The antibody conjugate of claim 1, wherein the antibody is a monoclonal antibody, including but not limited to anti-CD20 antibody, anti-CD19 antibody, anti-CD30 antibody, anti-EGFR antibody, anti-EGFRvIII antibody, anti-HER2 antibody, anti-HER3 antibody, anti-PSMA antibody, anti-VEGFR antibody, anti-PD-L1 antibody, anti-cMET antibody, anti-TGF-β antibody, anti-MUC1 antibody, and anti-Trop-2 antibody.

3. The antibody conjugate of claim 1, wherein the synthetic pathway of the hapten derivative is: 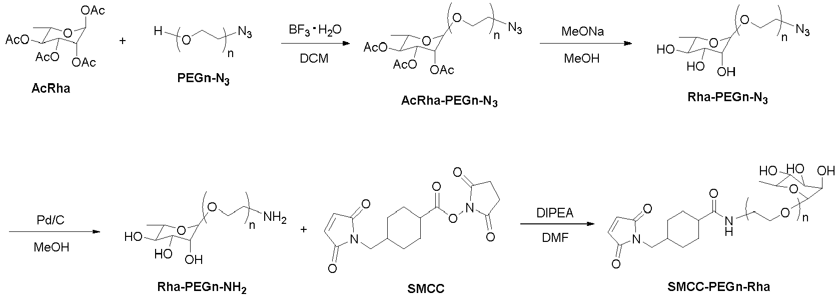 wherein n is a positive integer.

4. A pharmaceutical composition, comprising an antibody conjugate of claim 1, for use in the preparation of drug with enhanced immune effect function of antibody,
wherein the antibody conjugate is an antibody modified with a hapten derivative; wherein:
the hapten derivative is selected from any one hapten derivative of claim 1-2,
the antibody is selected from any one antibody of claim 1-2.

5. The method of claim 4, wherein the method for preparing an antibody conjugate by modifying the hapten derivative onto an antibody, comprising the following steps:
1) Desalting the antibody in a sodium borate reaction.
2) Adding a thiol-containing reducing agent TCEP and reacting under dark and shaking conditions.
3) After the reaction is completed, adding a 10-fold excess of the hapten derivative and quenching the reaction with cysteine.
4) Ultrafiltration to remove excess small molecules and obtain the corresponding antibody conjugate.
